# EUROPEAN PATENT APPLICATION

(11) **EP 3 758 015 A1**
(43) Date of publication of application: **30.12.2020**
(21) Application number: 19183046.2
(22) Date of filing: 27.06.2019
(51) Int. Cl.: G16H 30/40, G16H 40/60

(54) **DEVICE AT THE POINT OF IMAGING FOR INSTANT ADVICE ON CHOICES TO STREAMLINE IMAGING WORKFLOW**

(71) Applicant: Koninklijke Philips N.V., 5656 AG Eindhoven (NL)
(72) Inventor: Bystrov, Daniel, 5656 AE Eindhoven (NL); Young, Stewart, 5656 AE Eindhoven (NL); Rindt, Karsten, 5656 AE Eindhoven (NL); Sénégas, Julien, 5656 AE Eindhoven (NL); Poppe, Michaela, 5656 AE Eindhoven (NL); Burghardt, Sandra, 5656 AE Eindhoven (NL); Rohse, Thomas, 5656 AE Eindhoven (NL); Hawellek, Benjamin, 5656 AE Eindhoven (NL); Kurze, Christoph, 5656 AE Eindhoven (NL); von Berg, Jens, 5656 AE Eindhoven (NL)
(74) Representative: de Haan, Poul Erik

(57) **Abstract**

An imaging system(SYS), comprising a medical imaging apparatus (IA). The medical imaging apparatus comprises a detector (D) for acquiring a first image of a patient in an imaging session, and a display unit (DD) for displaying the first image on a screen. The system further comprises, distinct from the medical imaging apparatus (IA), a mobile image processing device (MIP). The mobile processing device (MIP) comprises an interface (IN) for receiving a representation of the first image, and an image analyzer (IAZ) configured to analyze the representation and, based on the analysis, to compute, during the imaging session, medical decision support information. The decision support information is displayed on an on-board display device (MD) of the mobile processing device (MIP).

## Description

### FIELD OF THE INVENTION

The invention relates to an image processing system, to the use of a mobile image processing device in said system, a mobile processing device, a method of image processing, a computer program element, and a computer readable medium.

### BACKGROUND OF THE INVENTION

Previously it was largely expert operators such as radiographers (x-ray, CT or MRI), sonographers (ultrasound), or nuclear medicine technicians (NM imaging) that operated medical imaging equipment. However, a new trend is emerging wherein less qualified staff is put in charge to perform examinations. This practice, without safeguarding, may lead to loss of clinical quality.

The operator (referred to herein as "the user") is responsible for performing a set of work-steps throughout the examination, including for example, depending on the modality and the specifics of equipment:
(i) patent positioning
(ii) adapt parameters of the imaging scan while the procedure progresses,
(iii) perform acquisition itself, and
(iv) review and post-process the resulting images at a console of the imaging equipment.

Once the imaging examination has been completed, subsequent steps in modern radiology workflows are typically organized such that the operator sends the images electronically to an image database (PACS) for storage, and simultaneously via a reading-worklist to another trained expert (medically-certified radiologist), for interpretation of the examination's findings. Depending upon a number of factors such as the urgency of the medical situation and the institution-specific organization of the workload, this interpretation often takes place in an asynchronous manner, meaning there is a significant time-delay between image acquisition and the image interpretation.

Artificial intelligence (AI) has the potential to compensate the lack of qualified personnel, while also improving clinical efficiency. AI systems are computer implemented systems. They are based on machine learning algorithms that have been pre-trained on training data to perform a task, such as assisting the user during the examination. Whilst such AI systems exit, these are usually integrated into a given imaging equipment or hospital IT infrastructure for a given medical facility. Furthermore, these AI systems may differ from facility to facility and may not be easy to operate or the AI output may not always be readily understood. Furthermore, some medical facilities may simply not have such AI systems at all, such as those in rural areas for example, or those in emerging markets.

### SUMMARY OF THE INVENTION

There may therefore be a need for systems and methods to address at least some of the above noted deficiencies.

The object of the present invention is solved by the subject matter of the independent claims where further embodiments are incorporated in the dependent claims. It should be noted that the following described aspect of the image processing system according to the invention equally applies to the use of the mobile image processing device in the system, to the mobile processing device, to the method of image processing, to the computer program element, and to the computer readable medium.

According to a first aspect of the invention there is provided an imaging system, comprising:
a medical imaging apparatus (also referred to herein as "imager") comprising: a detector for acquiring a first image of a patient in an imaging session; and a display unit for displaying the first image on a screen;
distinct from the medical imaging apparatus, a mobile image processing device comprising:
   an interface for receiving a representation of the first image;
   an image analyzer configured to analyze the representation and, based on the analysis, to compute, during the imaging session, medical decision support information, and
   an on-board display device for displaying the decision support information.

The mobile image processing device ("MIP") is preferably distinct and independent from the medical imaging apparatus. The interface is a universal one and affords interoperability with a range of different medical imaging apparatuses, even of different modality. The interface is independent in the sense that it is not embedded into the imaging equipment, and therefore the mobile device can be interfaced to an arbitrary imager. The MIP can be used as an add-on with existing imaging apparatuses. The MIP can be used at the point of imaging. Specifically, the analyzer is configured to compute the decision support information ("DSI") in real-time, that is, during the imaging session. The imaging session comprises the period of time during which the patient resides in or at the imaging apparatus or at least during which the patient is in an examination room where the imaging apparatus is present.

In embodiments, the interface of the mobile image processing device comprises an imaging component configured to capture during the imaging session the displayed first image as a second image, the said second image forming the said representation.

In other words, in this embodiments is based on direct imaging ("image of-image") of the displayed image. In other embodiments, the interface is arranged as NCF or bluetooth, if imaging apparatus is so equipped. Other embodiments still include LAN, WLAN, etc.

In embodiments, the decision support information includes and one or more of: ii) a recommended work flow in relation to the patient ii) an indication of an image quality in relation to the first image, ii) an indication on a medical finding, iii) a priority information.

In embodiments, the recommend work flow is in variance to a previously defined workflow envisaged for the said patient.

In embodiments, the indication on image quality includes an indication of one any one or more of: a) patient positioning, b) collimator setting, c) contrast, d) resolution, e) noise, f) artifact.

In embodiments, the image analyzer includes a pre-trained machine learning component.

In embodiments, the recommended work flow is put into effect automatically or after receiving a user instruction through a user interface of the mobile device.

In embodiments, the image analyzer is wholly integrated into the mobile device or wherein at least a part of the image analyzer is integrated into a remote device communicatively couplable to the mobile device through a communication network.

In embodiments, the mobile image processing device is a handheld device including any one of: i) a mobile phone, ii) a laptop computing device, iii) a tablet computer.

In another aspect, there is provided the mobile image processing device, when used in the system as per any one of the above mentioned embodiments.

In another aspect, there is provided a use of the mobile image processing device in a system as per any one of the above mentioned embodiments.

In another aspect there is provided a mobile image processing device including an imaging component capable of acquiring an image representing medical information in relation to a patient, and including an analyzer logic configured to compute decision support information in relation to the said patient based on the image, wherein the imaging component includes an image recognition module in cooperation with an auto-focus module of the imaging component, the recognition module configured to recognize at least one rectangular object in a field of view of the imaging component.

In embodiments, the analyzer logic is implemented in processor circuitry configured for parallel computing, for instance a multicore processor, a GPU or parts thereof.

The image analyzer may be included in a system-on-chip (SoC) circuitry.

Method of image processing, comprising the steps of:
by a detector of a medical imaging apparatus, acquiring a first image of a patient in an imaging session;
displaying the first image on a screen;
by a mobile image processing device distinct from the medical imaging apparatus, receiving a representation of the first image;
analyzing the representation and, based on the analysis, to compute, during the imaging session, medical decision support information, and
displaying the decision support information an on-board display device.

In another aspect, there is provided, a computer program element, which, when being executed by at least one processing unit, is adapted to cause the processing unit to perform the method.

In another aspect, there is provided a computer readable medium having stored thereon the program element.

*"user"* a referred to herein is medical personnel at least partly involved in an administrative or organizational manner in the imaging procedure.

*"patient"* is a person, or in veterinary settings, an animal (in particular a mammal), who is be imaged.

*"machine learning* (*"ML"*) *component"* is any computing unit or arrangement that implements a ML algorithm. An ML algorithm is capable of learning from examples (*"training data"*). The learning, that is, the performance by the ML component of a task measurable by a performance metric, generally improves with the training data. Some ML algorithms are based on an ML model that is adapted based on the training data.

### BRIEF DESCRIPTION OF THE DRAWINGS

Exemplary embodiments of the invention will now be described with reference to the following drawings, which are not to scale, wherein:
Fig. 1 shows a block diagram of an imaging arrangement;
Fig. 2 is a block diagram of a mobile image processing device as envisaged in embodiments and as may be used in the arrangement of Fig. 1;
Fig. 3 shows a use case of the mobile image processing device as envisaged in embodiments;
Fig. 4 shows a mobile image processing device in use in conjunction with a positioning device;
Fig. 5 shows various embodiments of a positioning device for a mobile image processing device;
Figs. 6-9 shows embodiments of communication networks in which the proposed mobile image processing device may be used;
Fig. 10 shows a flow chart of image processing; and
Fig. 11 shows a machine learning model.

### DETAILED DESCRIPTION OF THE EMBODIMENTS

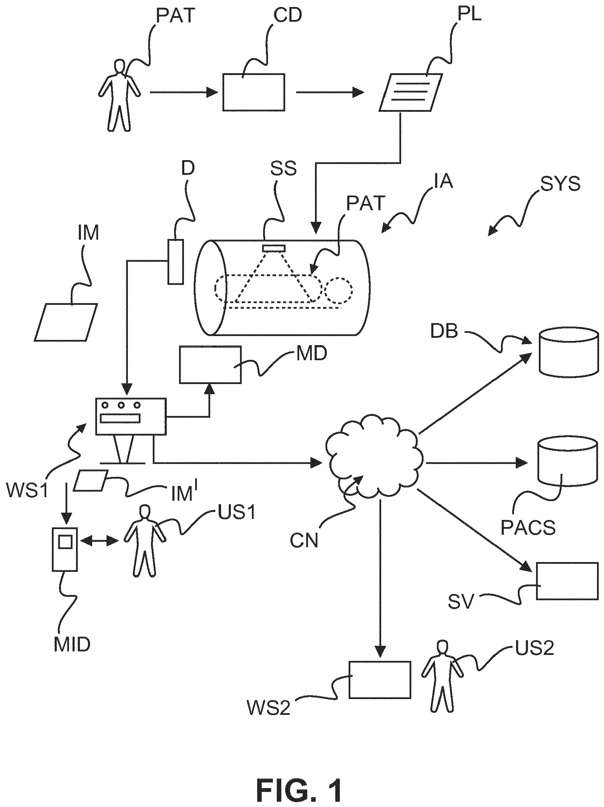

With reference to Fig. 1, this shows a schematic block diagram of an arrangement AR envisaged in medical or clinical set ups. However, the following description is not necessarily confined to medical fields.

In a medical facility, such as a GP practice, clinic, hospital or other, a patient PAT is checked in at a check-in desk CD. The patient PAT either already has a treatment plan PL assigned, or such is assigned at check-in CD. The treatment plan PL prescribes a number of medical procedures to be performed in respect of the patient. One step of such procedure may include imaging for diagnostic or therapeutic purposes. Imaging can be done by an imaging apparatus IA.

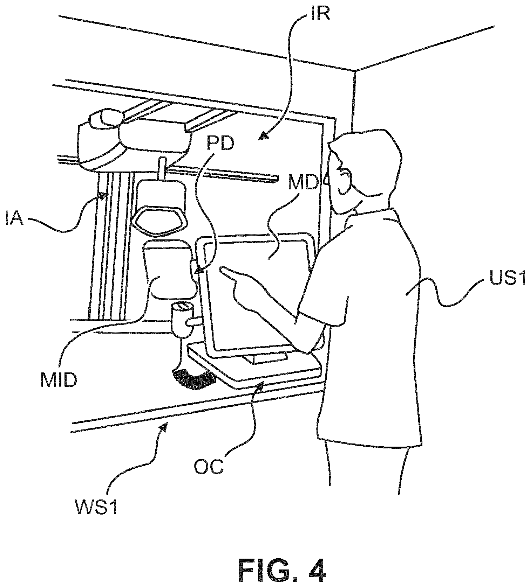

The imaging apparatus IA may be of any modality such as transmission or emission imaging. Transmission imaging includes for instance x-ray based imaging carried out with a CT scanner or other. Magnetic resonance imaging MRI is also envisaged and so is ultrasound imaging. Emission imaging includes PET/SPECT and other nuclear medicine modalities. To perform the imaging, the patient PAT is led into an imaging room IR (see Fig 4) where the imaging apparatus IA is situated.

During an imaging session, images IM are required of the patient. The images IM are preferably in digital form and may assist a physician in diagnosis. In order to facilitate correct imaging during the imaging session the arrangement includes a computerized system SYS to support imaging operation of the image IA. The user US1 may not necessarily be a physician with a medical degree but may instead be a medical technician or a user of lesser training. The system SYS promotes safe and correct use of the imager, even for staff with low level medical skills, semi-skilled or trained-on-the-job, etc.

The system SYS includes, preferably mobile, image processing device MID which can be operated by the user US1 to assist him or her in the task of correctly and safely acquiring the images of the patient PAT in the imaging session. The device MID, referred to herein as the "mobile device" MID, is distinct and separate from the imaging apparatus IA. As will be explored more fully below, the mobile device MID includes a universal interface IF through which a copy IM' of an image acquired by the imaging apparatus, referred to herein as the "source image" IM, can be received.

The mobile device MID includes in particular an image analyzer IAZ component that allows analyzing the copy image IM, to obtain decision support information which can be displayed on an on-board display OD of the mobile device MID. This information can assist the user US1 in assessing, for example, whether the source image IM is of sufficient quality. The displayed information may include suggestions for further steps, which may include a suggestion for an imaging retake, if the quality is found of inferior quality. In addition or instead, the information may indicate the presence of a medical condition and may further include suggestions for changing the pre-assigned plan PL. Based on the analysis performed by the mobile device MI, the plan PL may be adapted or changed as will be explained more fully below.

Depending on the displayed decision support information, the user US1 may decide to forward the source image IM through a hospital communication network CN to an image repository such as a PACS. The hospital information infrastructure HIS may include other data bases DB, servers SV, or other workstations WS2 of other users US2 which can be accessed through the communication network CN. In addition or instead of forwarding the source image to a repository, this may be forwarded direct to a physician US2 at a workstation WS2 for interpretation or "reading" to establish a diagnosis for instance. Alternatively, the physician may retrieve the imager from the PACS. As mentioned, the technician US1 is in general not involved in the interpretation of imagery. This task is left to physicians US2 with a medical degree who have training in image reading. The imager IA user US1, supported by the mobile device MID, can focus his or her attention solely to technical considerations in acquiring the source image IM correctly, of sufficient quality and according to protocol. The physician US2 can then rest assured that the correct image has been acquired and he can focus his or her attention to interpreting the imagery and not be bothered by technical aspects of image acquisition.

Turning now in more detail, to the envisaged arrangement AR, and with continued reference to Fig. 1, the imaging apparatus IA includes in general a signal source SS. During image acquisition in the imaging session, the signal source SS emits an interrogating signal which interacts with tissue in the patient. As a result of the interaction with the tissue, the signal is modified. The so modified signal is then detected by a detector unit D. Acquisition circuitry converts the detected signals, such as intensities, into a digital image, the source image IM.

Adjustment of imaging parameters and overall control of the imaging apparatus throughout image acquisition is performed by technical user US1 from an operator console OC that may include a stationary computing device. The operator console OC may be positioned in the same room IR as the imager IA or may be situated in a separate room. The operator console is communicatively coupled to a display device, referred to herein as the monitor MD, associated with the operator console OC and the imager IA. The acquired image is forwarded by the acquisition circuitry to a computing unit WS1, a workstation, in the operator console OC operable by the user US1. The operator console may be communicatively coupled into the HIS through network CN.

The acquired source image IM may be displayed on the main monitor MD. This allows the user US1 to roughly ascertain whether the source image is correct. Previously, if the user US1 felt the image is correct the source image, or a plurality of source images such as is acquired in a time series (a motion picture), may be forwarded into the hospital information structure through the communication network to its intended destination such as the PACS or maybe directly forwarded to the physician US2 at his or her workstation WS2.

As proposed herein, before the user US1 makes the decision to forward the source images IM into the hospital infrastructure, user US1 may use the mobile device MID to analyze the source image to establish image quality and/or a medical finding. The analysis is done by the mobile device MID acquiring a copy IM' of the source image IM and then analyzing the copy image IM'. Advantageously, as proposed herein, the mobile device MID is not integrated into, or "bundled up", with the hospital information structure or with the imaging apparatus IA or operator console or work station. Rather, the mobile imaging processing device MID is a separate, independent, standalone unit that is preferably envisaged to be able to analyze the received copy IM' on its own to compute the decision information and to display the same on its own display OD for the user US1. This is advantageous as not all medical facilities have image quality assessment functionalities provided at the point of imaging. Specifically, at a given imaging apparatus at a given department or facility, the image quality assessment functionality may or may not be integrated into the workstation WS1 or into the operator console. The user US1 may be on circuit, that is, may be assigned to different departments of the same medical hospital or may indeed be assigned to work at different medical facilities in a geographical region, and is hence asked to operate a range of different medical imaging equipment from different manufacturers and/or across different modalities. In this situation, the user US1 can use consistently his or her own mobile device MID to reliably analyze the acquired imagery, independently from the given infrastructure. This ensures consistent quality of care across facilities.

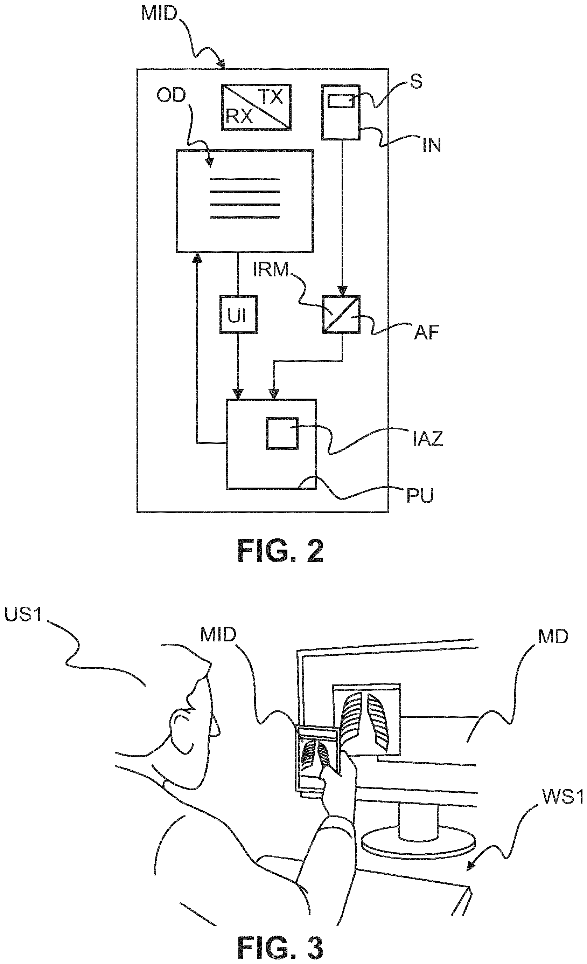

Reference is now made to the block diagram of Fig. 2 which furnishes more details of the envisaged mobile image processing device MID. As mentioned, the mobile device MID includes a universal interface IN that allows to receive the copy IM' no matter the given imaging infrastructure.

In one embodiment, the universal interface IN is arranged as a camera with an image sensor S. The mobile device MID may be arranged as a smart phone, a tablet, a laptop, notebook or any other computing device with integrated camera.

The mobile device MID has its own onboard display OD. On this display the acquired copy IM' may be displayed as required. In addition or instead, the decision information provided by the image analyzer IAZ may be displayed on the onboard display device OD.

The image analyzer IAZ may be driven by artificial intelligence. In particular, the image analyzer IAZ may be included as a pre-trained machine learning component or model. The image analyzer IAZ may be run on a processing unit of the mobile device MID. The processing unit may include general purpose circuity and/or dedicated computing circuitry such as a GPU or may be a dedicated core of a multi-core multi-processor. Preferably, the processing unit is configured for parallel computing. This is in particular advantageous if the underlying machine learning model is a neural network such as a convolutional network. Such types of machine learning models can be efficiently implemented by vector, matrix or tensor multiplications. Such types of computations can be accelerated in a parallel computing infrastructure.

The mobile device MID may further comprise communication equipment including a transmitter TX and a receiver RX. The communication equipment allows connecting with the hospital network CN. Envisaged communication capabilities include any one or more of Wi-Fi, radio communication, Bluetooth, NFC or others.

In a preferred embodiment the mobile device is configured for an "image-of-an image" functionality to acquire a copy IM' of the source image IM. More specifically, the user US1, after the source IM has been acquired and is displayed on the main display MD, operates the mobile device MID to capture an image of the source image IM as displayed on the main display MD. The so captured image forms the copy image IM'.

So as to better aid the user US1 in capturing this copy image IM', the image sensor S may be coupled to an auto focus AF functionality that automatically adjusts focus and/or exposure. Preferably still, the auto focus AF is coupled to an image recognition module IRM that assists the user US1 in capturing the copy image IM' with good focus on the source image IM as displayed on main monitor MD. To this end, the image recognition module IRM is configured to search the field of view for square or rectangular objects as such is the expected shape of the source image when displayed on the main monitor MD or the shape of the main display MD itself. During focusing with automatic object shape recognition, an outline of the captured object may be indicated in the field of view to assist the user US1. For instance, the outlines of a square or rectangle that represents the borders of the main display MD as represented in the current field of view or the borders of the source image IM itself as currently displayed on the main display may be visualized.

Once the correct object is in focus, the user requests image capture by operating a virtual or real shutter button UI. The captured image, the copy IM', is stored in an internal memory of the mobile device MID. The captured copy image IM's is forwarded for analysis to the imager analyzer IAZ. In order to exclude irrelevant information, the captured image may be automatically cropped before analysis so that the remaining pixel information represents solely medical information as per the source image IM.

The resolution of the copy IM' is in general lower than that of the source image IM and is dictated by the resolution capabilities of the image sensor S. To suitably factor in this drop-in resolution, the mobile device may include a setting menu that allows the user to input the native resolution of the source image. The resolution capability of the sensor and hence the resolution of the copy of image IM' may be automatically obtained or may be provided by the user. Based on this data, that is, the two resolutions or a ratio thereof, the image analyzer IAZ can factor in the drop-in resolution when analyzing the copy image IM'.

Other settings that the user may be able to specify may include the purpose of the imaging, in particular, a specification of the anatomy of interest such as chest, head, arm, leg or abdomen. The user may also input certain general patient characteristics of the patient such as sex, age, weight if available. Preferably, the on-board display accepts touch screen input. A user interface UI, such as graphical UI, may be displayed on the on-board screen, through which the user can apply or access the above described settings.

The image analyzer IAZ analyses the image preferably in two stages. In the first stage the image quality such as resolution, the correct collimator settings (if any), etc., is established. Image contrast may also be analyzed. Once the image quality satisfies certain predefined standards, the image may be further analyzed to establish a medical condition. If a medical condition is found, this may be flagged up on the onboard display OD preferably, with a prioritization level. The prior level may include a designation for "low", "medium" or "high" priority and/or a name of the medical condition. Finer or coarser priority level graduation may be used instead. For instance, if presence of an infectious disease, such as tuberculosis, is established, this may be flagged up as an instance of high urgency. If no medical condition is found, a confirmatory indication may be displayed such as an "OK" or there is simply no indication. In addition or instead, an indication for the image quality is displayed so as to indicate to the user whether or not the current IQ satisfies the predefined IQ criteria. The predefined IQ criteria may be user configurable.

The decision support information computed by the image analyzer may hence include any one or more of the following: IQ, medical finding and/or in associated priority level. In addition, or instead, if a medical condition is found, a related workflow may be suggested and displayed. This suggested workflow may be different from the currently assigned plan PL. If the user accepts the proposed workflow changes, the user may operate the user interface UI to initiate and register the changed plan PL'. This may be done by the mobile device connecting into the network CN and sending an appropriate message to the check-in desk CD or to the responsible physician US2, etc. If the IQ is found by the IAZ to be deficient, a retake may be proposed, optionally with a suggestion for updated imaging parameters. The user US1 may then accept the retake using the UI, and a suitably formatted message is sent to the operating console OC to adjust the imaging parameters and/or initiate the image retake.

The above described functionalities of the mobile device MID may be implemented by installing a software in a generic handheld device with imaging capability. This can be done by the user US1 downloading an "app" from a dispensing server, an "app store", onto their generic handheld device.

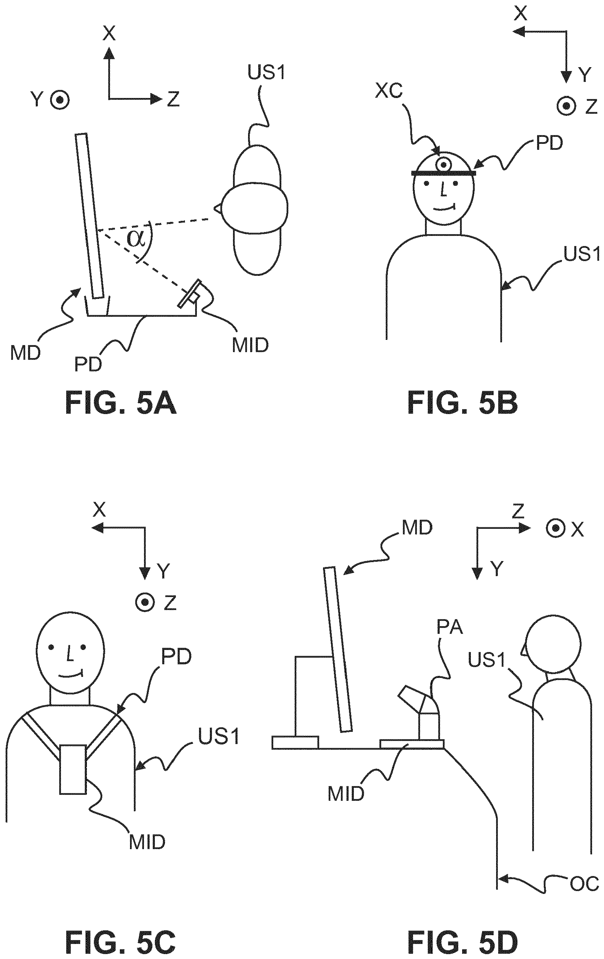

In order to still better assist the user US1 in capturing the copy image IM' of the source image, a position device PD may be supplied with the mobile device MID as will now be discussed with reference to embodiments in Fig. 4 and Figs. 5A)-5D). However, such a position device PD is optional, and the user may instead simply hold the device in front of the main screen MD when capturing the image IM', such as shown in the schematic use case in Fig. 3.

Referring first to Fig. 4, this shows another positioning device PD that allows the user to place the mobile device MID side by side to the main display. The positioning device thus includes a cradle to receive the mobile device, with a clip or attachment means with which the cradle can be attached to, for instance, the side or top edge of the main monitor MD. The user US1 can hence easily operate the mobile device MID and the console CO hands free, with a clear view of the main display MD and the on-board display OD of the mobile device MID.

Referring now to Fig. 5A, this shows a further embodiment of the position device PD in plan view. This embodiment may include an arm with a clip or other attachment means at one of its ends. The arm is attachable via the attachment means to an edge of the main monitor MD. The position device PD terminates in the other end in a preferably articulated cradle to receive the imaging device MID. Using such a position device allows the user hands free operation and the image acquisition may be triggered by voice recognition with the user making a predefined utterance such as 'capture' to operate the mobile device MID to capture the image in the current field of view. The image analyzer may include logic that accounts for the annular deviation α which is expected when the mobile device captures the image not from directly in front but from an angle at the said angle α. The angle may be adjusted thanks to the articulation of the cradle.

Although the camera device is preferably fully integrated into the mobile device, this may not necessarily be so in all embodiments, where there is an external camera device XC that is communicatively coupled through Bluetooth or any other wireless or indeed wired communication means with the mobile device MID as shown in Fig 5B. In this embodiment, the external camera may be attached via a headband PD to the user's forehead. This arrangement allows capturing imaging in full frontal head-on view rather than at an angle as in Fig. 4A. Again, image acquisition of the copy IM' may be initiated by voice command or by the user using a real or virtual shutter button provided by the mobile device MID. Alternatively, but not shown, the external camera XC may be positioned on a small tripod in front of the monitor, suitably aligned.

It is also the embodiment of the positioning device PD in Fig. 5C that allows capturing images head-on. In this embodiment, this is achieved by using a neckband or lanyard around the user's neck with the mobile device pending therefrom on a connector. The mobile device, in use, may then be positioned on the user's US1 chest to allow for acquiring images in frontal view, in particular when using a front-facing camera of the device MID, if any. As opposed to a rear-facing camera, a front-facing ("selfie"-) camera is one that can capture imagery of an object with the device MID's user interface or on-board display OD directed towards said object.

In another embodiment as per Fig 5D, there is provided a periscopic adaptor PA which is attached to the viewfinder of the integrated camera of the mobile device MID. The attachment may be via a suction cup for instance. The periscopic adaptor allows diverting the optical path at an angle. During imaging, the mobile device, with the viewfinder facing upwards, may be lying flat on a surface such as on a ledge or working platform of the operator console.

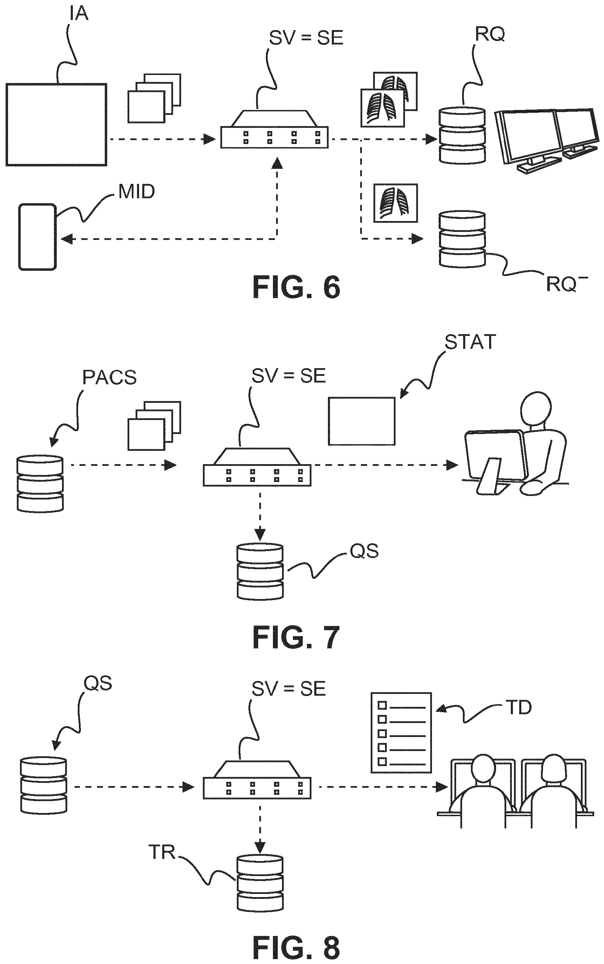

Referring now to Fig. 6, this shows one example of how the mobile device may be used in hospital information technology infrastructure. Whilst the image analyzer IAZ may be fully integrated into the mobile device MID, alternative embodiments are also envisaged where at least a part or all of the image analyzing capability is outsourced to a "smart engine" SE which may be arranged as a functionality in one of the servers SV of the communication network CN, or indeed in a remote server not part of the network but connectable thereto. For instance, the user after installing the above mentioned app, may purchase a subscription to access a Cloud based image analyzer functionality.

Whilst the mobile device MID as such is independent of the given hospital infrastructure or imager IA, a certain level of integration through standardize interfaces such as Bluetooth, LAN, WLAN or other may still be possible so that the user may request direct from the mobile device the forwarding of the source images IM through the hospital network to the PACS, other user US, etc., based on the received decision support information.

With further reference to Fig 6, in embodiments, depending on the priority which is assigned to analyzed copy image IM', a plurality of different reading queue RQ and RQ⁻ can be established. The counterpart source images IM are then divided into those queues. Specifically, source imagery that are awarded a higher priority than others based on the analysis of their counterpart copy image, are forwarded to a higher priority reading queue RQ while those of lesser urgency are relegated to a second image queue for less urgent imageries RQ⁻. This allows the image reader US2 to better manage their workload.

Specifically, based on the analysis of the copy images by the smart engine, the counterpart source images IM are routed through the network CN from the imager IA to the PACS. This routing may be requested by the user from the mobile device MID, or the user may request this from the work-station WS1 or console OC. The Smart Engine SE analyzes the images, and forwards the decision support information to proposed device MID. The user US1 may then authorize, via confirmatory feedback from the device MID to forward the source images from the imager to the PACS, into the respective queue RQ and RQ⁻, using an appropriate AE (application entity) title.

The Smart Engine may include software components that run on appropriate hardware in the local IT infrastructure SV. The network connection to the proposed device MID could be implemented using LAN or WLAN or other, as required. In an embodiment, there is a feedback communication channel that enables the radiologist US2 to provide image quality feedback at the time of image reading, which may occur significantly after the actual image acquisition.

The feedback information and/or the decision support information may be gathered and stored as statistical information in the same or a separate database QS. The statistical information STAT represents an overall picture of IQ (image quality) of imagery produced at the relevant medical facility or group of such facilities. This aspect is further illustrated in Fig 7. Fig 7 provides a schematic overview of the integration of the Smart Engine with a database of image quality statistics, for the purpose of retrospective analysis of the image quality status over a specified time period. Fig 7 illustrates how the proposed device MID may be integrated into a larger system of image quality monitoring, enabling the retrospective analysis of image quality status, for example by administrative radiology staff. Such an assessment could be evaluated both as a baseline assessment at the beginning of quality improvement initiatives, as well as to monitor image quality on an on-going basis. Images are retrieved from PACS, and quality measurements made on the Smart Engine are stored in a database of quality statistics. Intermediate results of the statistical analysis may be forwarded, automatically, once or periodically, or on user request, to the mobile device MID and may be displayed on the on-board display OD. A web server may be used to host the Smart Engine, together with a database management system for the statistical data STAT.

Fig. 8 is a schematic overview of a network with integration of the Smart Engine in a user-adaptive training situation. Image quality information and related statistical information STAT is used for the purpose of retrospective analysis of the image quality status over a specified time-period. User-adaptive training may be implemented. An analysis of the image quality statistics identifies the individual training recommendations for specific users US1, which can be deployed via a recommended system. A quality statistics database QS hosted by Smart Engine server is connected with user-specific training content TD. Users US1 can use a standard office PC to start, in embodiments, a client, such as a web-based thin client, to access the tailored content TD. The mobile device MID may be used with the thin-client as an app to access the training content. Executed training sessions with results are stored in training records database. The system comprises a training user interface which allows retrieving any one or more of a recommendation (eg, from a supervisor or a more experienced colleague who has reviewed user-specific statistics), a training framework, and the training content. In embodiments, a web-client based reporting application may be used to access this information. The training content may be stored on Smart Engine SE. The content may be customizable, e.g. by an administrator.

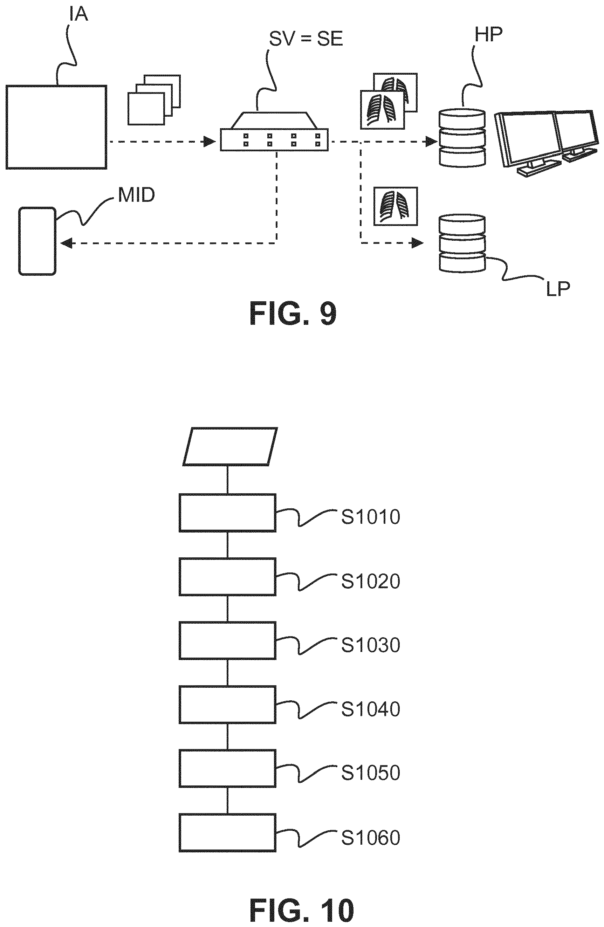

Fig. 9 shows a schematic overview of a network with integration of the smart Engine to deploy a clinical decision support system. The proposed device MID is used to display the results of an analysis of the images IM (transmitted e.g. via LAN) or copy images IM' via a clinical decision support application which may be run by the smart engine. Specifically, the proposed device MID may be used to display results of the clinical decision support at the point of imaging. The copy images IM' or the acquired source images IM are sent to the Smart Engine server SE and analyzed by Clinical Decision Support application. Instant feedback is sent to the mobile device MID for the attention of the user US1, in particular for high priority images HP where an immediate work flow step is required. For example, if an infectious disease is detected in the image, the patient must immediately be isolated from other patients in the hospital to prevent spreading. Other, low priority images LP , are forwarded to the PACS and stored in the appropriate folder (AE title).

It will be understood that principles of the embodiments in Figs. 6-9, such as the reading queues, the statistical evaluation etc., may also be implemented in embodiments without remote smart engine, that is, in embodiments where the image analyzer is wholly or partly implemented on the mobile device MID itself.

Reference is now made to Fig. 10, which shows a flow chart of a method of image processing that relates to the system described above. However, it will be appreciated that the below described method is not necessarily tied to the above described system. The following method may hence be understood as a teaching in its own right.

At step S1010 a first digital image, referred to herein as the source image, of a patient is acquired in an imaging session by an imaging apparatus.

At an optional step S1020 the source image is displayed on a stationery screen of the first display unit.

At step S1030 a second digital representation (a "copy" image) of the source image is received at an image processing device. The image processing device is preferably mobile, such as a handheld device, and is independent and distinct from stationary computing units such as a workstation and/or an operator console coupled to the medical imaging apparatus.

At step S1040 this second image, the copy image, is analyzed to compute during the imaging session medical support information in relation to the source image.

At step S1050 the computed medical decision support information is displayed on an onboard display device of the mobile processing device.

At an optional step S1060, a user response is received through a user interface of the mobile device. The user response represents a requested action in connection with the displayed decision support information. The user may for instance request one or more of the suggested workflow steps to be performed in relation to the patient. The requested workflow step(s), which may differ from a pre-assign workflow, may include an image retake, or a referral to specialist, or booking of other medical equipment in the instant or another medical facility.

In a further step S1070, the user request is initiated by sending a corresponding message across the network to a recipient, e.g. to the check-in desk CD or to device associated with a physician.

Alternatively, the recommended one or more work steps of are effected automatically without user confirmation through an interface. In this embodiments, upon analysis of the copy image(s), the changed workflow is initiated by sending respective messages or control signals to the relevant network actors, comprising the imager IA, the hospital IT infrastructure, etc.

In embodiments, the copy image is captured by an imaging component of the mobile device. The copy image is an "image of-an-image", in other words, is an image representation of the source image, acquired by the imaging component whilst the source image is displayed on a main display device associated with the imaging apparatus.

The imaging component is preferably integrated into the mobile imaging device, but an external imaging component may be used instead connectable to the mobile device. Instead of this "image-of-an image" scheme, a copy of the source image may be forwarded to the mobile imaging device through other interface means, such as NFC, Wi-Fi, attachment to email or text message, or by Bluetooth transmission.

The computed decision support information includes one or more of: a recommended workflow in relation to the patient, an indication of the image quality of the source image and an indication of medical findings in relation to the patient, such as a medical condition and preferably associated priority information. The priority information represents the urgency of the medical finding.

Preferably the computing of the decision support information is done in a two-stage sequential processing flow. In a first stage, the image quality is established. If the image quality is found to be sufficient, only then is the imagery analyzed for a medical finding and/or workflow suggestions. The workflow computed based on the analyzed image may differ from a workflow originally associated with the patient at check-in for instance. This change in workflow may be required for instance if an unexpected medical condition is detected in the image that was not previously envisaged by the original workflow. For instance, if the patient is to receive a cancer treatment of a certain organ, such as the liver, a certain workflow is envisaged. However, if the analysis of the copy image accidentally reveals that the patient is in fact suffering from pneumonia, the workflow needs to be changed to first treat pneumonia before proceeding with the cancer treatment.

The image quality analysis may include an assessment of patient positioning, collimator setting (if any), contrast, resolution, image noise or artifacts. Some or all of these factors may be considered and represented as a single image quality score in a suitable metric or each factor is measured by a separate score in a different metric. If the image quality is found to be of sufficient quality in embodiments no further display is effected on the onboard screen of the mobile device. Alternatively, and preferably, a suggestive graphical indication is given when the image quality is deemed sufficient. For instance, a suggestive "tick" symbol may be displayed in an apt coloring scheme, such as green or otherwise. If the image quality is found to be insufficient, this is also indicated on the onboard display in suggestive symbology such as a red cross or otherwise. If a medical condition is found, this is indicated by a suitable textual or other symbol on the onboard display of the mobile display device. A recommended workflow based on the finding may also be displayed in addition or instead.

In embodiments the user interface of the mobile device may be configured to receive a user input through a user interface. In response to a user input so received, the possibly proposed workflow may then be initiated by sending a suitable message to this effect through the communication network and onwards to the patient registry CD. In addition or instead, a message may be sent with the findings to a second user US2, such as responsible physician, to alert same to attend to the patient.

The decision support information is preferably provided within real time after representation of the source image is received at the mobile device. Particularly, the outcome of the analysis that is the decision support information is made available within seconds or fractions thereof. The computations required for the analysis may be wholly performed by a processing unit of the mobile device or may be partly or wholly outsourced to the external remote server with more powerful processing capability.

In embodiments the recommended workflow may include recommendation to retake the image based on the analysis. The technician US1 can then decide to follow this advice. Because of the real-time availability of the decision support information, user can attend to this immediately and retake the image whilst the patient is still in or at the imaging apparatus during the imaging session. Unnecessarily sending across of a deficient image through the network into the hospital information infrastructure, such as the PACS, can be avoided. This allows reducing network traffic and wasting of memory space.

In embodiments the analysis step S1040 is based on a pre-trained machine learning model. The machine learning model has been pre-trained on historic patient data retrievable from image repositories from the same hospitals or other hospitals. Preferably, a supervised learning scheme is used wherein the historic imagery is pre-labeled by experienced clinicians. Labeling provides target data that includes any one or more of an indication on the medical condition present in the historical imagery, an indication on the proposed workflow, and an indication whether the image quality is deemed sufficient.

Training of the machine learning component may include the following steps of receiving the training data, applying a machine learning algorithm to the training data, in one or more iterations. As a result of this application the pre-trained model is then obtained which can then be used in deployment. In deployment, new data, e.g. a copy image IM not from the training set, can be applied to the pre-trained model to obtain the desired decision support information for this new data.

The source image as displayed and capture may not necessarily be a single still image, but there may be a plurality of sequentially displayed source images, a motion picture or video that is. All the above and below is of equal application to such videos or motion pictures.

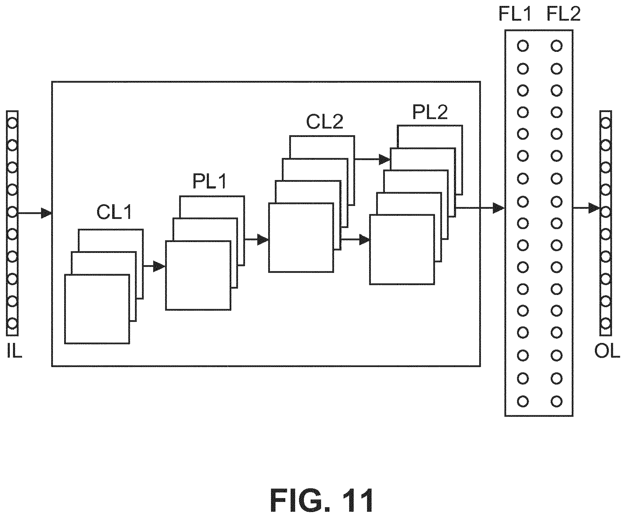

Reference is now made to Fig. 11 where a neural-network model is shown as may be used in embodiments. However, other machine learning techniques such as support vector machines, decision trees or other may be used instead of neural networks. Having said that, neural networks, in particular convolutional networks, have been found to be of particular benefit especially in relation to image data.

Specifically, Fig. 11 is a schematic diagram of a convolutional neural-network CNN. A fully configured NN as obtained after training (to be described more fully below) may be thought as representation of an approximation of a latent mapping between two spaces, the images and the space of any one or more of image quality metrics, medical findings and treatment plans. These spaces can be represented as points in a potentially high dimensional space, such as an image being a matrix of *N x N*, with *N* being the number of pixels. The IQ metrics, the Medical findings and treatment plane can be similarly encoded as vectors, matrices or tensors. For example, a work flow may be implemented as a matrix or vector structure, with each entry representing a work flow step. The learning task may be one or more of classification and/or regression. The input space of images may include 4D matrices to represent a time series of matrices, an hence a video sequence.

A suitable trained machine learning model or component attempts to approximate this mapping. The approximation may be achieved in a learning or training process where parameters, itself forming a high dimensional space, are adjusted in an optimization scheme based on training data.

In yet more detail, the machine learning component may be realized as neural-network ("NN"), in particular a convolutional neuro-network ("CNN"). With continued reference to Fig. 11, this shows in more detail a CNN architecture as envisaged herein in embodiments.

The CNN is operable in two modes: "training mode/phase" and "deployment mode/phase". In training mode, an initial model of the CNN is trained based on a set of training data to produce a trained CNN model. In deployment mode, the pre-trained CNN model is fed with non-training, new data, to operate during normal use. The training mode may be a one-off operation or this is continued in repeated training phases to enhance performance. All that has been said so far in relation to the two modes is applicable to any kind of machine learning algorithms and is not restricted to CNNs or, for that matter, NNs.

The CNN comprises a set of interconnected nodes organized in layers. The CNN includes an output layer OL and an input layer IL. The input layer IL may be a matrix whose size (rows and columns) matches that of the training input image. The output layer OL may be a vector or matrix with size matching the size chosen for the image quality metrics, medical findings and treatment plans.

The CNN has preferably a deep learning architecture, that is, in between the OL and IL there is at least one, preferably two or more, hidden layers. Hidden layers may include one or more convolutional layers CL1, CL2 ("CL") and/or one or more pooling layers PL1, PL2 ("PL") and/or one or more fully connected layer FL1, FL2 ("FL"). CLs are not fully connected and/or connections from CL to a next layer may vary but are in generally fixed in FLs.

Nodes are associated with numbers, called "weights", that represent how the node responds to input from earlier nodes in a preceding layer.

The set of all weights defines a configuration of the CNN. In the learning phase, an initial configuration is adjusted based on the training data using a learning algorithm such as forward-backward ("*FB*")-propagation or other optimization schemes, or other gradient descent methods. Gradients are taken with respect of the parameters of the objective function.

The training mode is preferably supervised, that is, is based on annotated training data. Annotated training data includes pairs or training data items. For each pair, one item is the training input data and the other item is target training data known a priori to be correctly associated with its training input data item. This association defines the annotation and is preferably provided by a human expert. The training pair includes historic imagery as training input data, and associated with each training image, is target of label for any one or more of: IQ indication, indication of medical finding represented by that image, indication of a priority level, indication of work flow step(s) called for given the image.

In training mode, preferably multiple such pairs are applied to the input layer to propagate through the CNN until an output emerges at OL. Initially, the output is in general different from the target. During the optimization, the initial configuration is readjusted so as to achieve a good match between input training data and their respective target for all pairs. The match is measured by way of a similarity measure which can be formulated in terms of on objective function, or cost function. The aim is to adjust the parameters to incur low cost, that is, a good match.

More specifically, in the NN model, the input training data items are applied to the input layer (IL) and passed through a cascaded group(s) of convolutional layers CL1, CL2 and possibly one or more pooling layers PL1, PL2, and are finally passed to one or more fully connected layers. The convolutional module is responsible for feature based learning (e.g. identifying features in the patient characteristics and context data, etc.), while the fully connected layers are responsible for more abstract learning, for instance, the impact of the features on the treatment. The output layer OL includes the output data that represents the estimates for the respective targets.

The exact grouping and order of the layers as per Fig 11 is but one exemplary embodiment, and other groupings and order of layers are also envisaged in different embodiments. Also, the number of layers of each type (that is, any one of CL, FL, PL) may differ from the arrangement shown in Fig 11. The depth of the CNN may also differ from the one shown in Fig 11. All that has been said above is of equal application to other NNs envisaged herein, such as fully connected classical perceptron type NN, deep or not, and recurrent NNs, or others. In variance to the above, unsupervised learning or reinforced leaning schemes may also be envisaged in different embodiments.

The annotated (labelled) training data, as envisaged herein may need to be reformatted into structured form. As mentioned, the annotated training data may be arranged as vectors or matrices or tensor (arrays of dimension higher than 2). This reformatting may be done by a data pre-processor module (not shown), such as scripting program or filter that runs through patient records of the HIS of the current facility to pull up a set of patient characteristics.

The training data sets are applied to the an initially configured CNN and is then processed according to a learning algorithm such as the FB-propagation algorithm as mentioned before. At the end of the training phase, the so pre-trained CNN may then be used in deployment phase to compute the decision support information for new data, that is, newly acquired copy images not present in the training data.

Some or all of the above mentioned steps may be implemented in hardware, in software or in a combination thereof. Implementation in hardware may include a suitably programmed FPGA (field-programmable-gate-array) or a hardwired IC chip. For good responsiveness and high throughput, multi-core processors such as GPU or TPU or similar may be used to implement the above described training and deployment of the machine learning model, in particular for NNs.

One or more features disclosed herein may be configured or implemented as/with circuitry encoded within a computer-readable medium, and/or combinations thereof. Circuitry may include discrete and/or integrated circuitry, application specific integrated circuitry (ASIC), a system-on-a-chip (SOC), and combinations thereof., a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An imaging system(SYS), comprising:
a medical imaging apparatus (IA) comprising: a detector (D) for acquiring a first image of a patient in an imaging session; and a display unit (DD) for displaying the first image on a screen;
distinct from the medical imaging apparatus (IA), a mobile image processing device (MIP) comprising:
an interface (IN) for receiving a representation of the first image;
an image analyzer (IAZ) configured to analyze the representation and, based on the analysis, to compute, during the imaging session, medical decision support information, and
an on-board display device (MD) for displaying the decision support information.

2. Image processing system of claim 1, wherein the interface (IN) of the mobile image processing device (MIP) comprises an imaging component (S) configured to capture during the imaging session the displayed first image as a second image, the said second image forming the said representation.

3. Image processing system of any one of claims 1 or 2, wherein the decision support information includes and one or more of: ii) a recommended work flow in relation to the patient ii) an indication of an image quality in relation to the first image, ii) an indication on a medical finding, iii) a priority information.

4. Image processing system of claim 3, wherein the recommend work flow is in variance to a previously defined workflow envisaged for the said patient.

5. Image processing system of any one of claims 1-4, wherein the indication on image quality includes an indication of one any one or more of: a) patient positioning, b) collimator setting, c) contrast, d) resolution, e) noise, f) artifact.

6. An image processing system of any one of the previous claims, wherein the image analyzer (IAZ) includes a pre-trained machine learning component.

7. An image processing system of any one of claims 3-6, wherein the recommended work flow is put into effect automatically or after receiving a user instruction through a user interface (UI) of the mobile device.

8. An image processing system of any one the previous claims, wherein the image analyzer (IAZ) is wholly integrated into the mobile device (MIP) or wherein at least a part of the image analyzer is integrated into a remote device (SE) communicatively couplable to the mobile device (MIP) through a communication network (CN).

9. An image processing system of any one of previous claims, wherein the mobile image processing device (MIP) is a handheld device including any one of: i) a mobile phone, ii) a laptop computing device, iii) a tablet computer.

10. The mobile image processing device (MIP), when used in a system (SYS) as per any one of the previous claims.

11. A mobile image processing device (MIP) including an imaging component (S) capable of acquiring an image representing medical information in relation to a patient, and including an analyzer logic (IAZ) configured to compute decision support information in relation to the said patient based on the image, wherein the imaging component (S) includes an image recognition module (IRM) in cooperation with an auto-focus (AF) module of the imaging component (S), the recognition module configured to recognize at least one rectangular object in a field of view of the imaging component (S).

12. Mobile image processing device of claim 11, wherein the analyzer logic is implemented in processor circuitry configured for parallel computing.

13. Method of image processing, comprising the steps of:
by a detector (D) of a medical imaging apparatus (IA), acquiring (S1010) a first image of a patient in an imaging session;
displaying (S1020) the first image on a screen;
by a mobile image processing device (MIP) distinct from the medical imaging apparatus (IA), receiving (S 1030) a representation of the first image;
analyzing (S1040) the representation and, based on the analysis, to compute, during the imaging session, medical decision support information, and
displaying (S1050) the decision support information an on-board display device (MD).

14. A computer program element, which, when being executed by at least one processing unit (PU), is adapted to cause the processing unit (PU) to perform the method as per claim 13.

15. A computer readable medium having stored thereon the program element of claim 14.
